(19) Europäisches Patentamt
European Patent Office
Office européen des brevets

(11) **EP 2 927 679 B1**

(12) **EUROPÄISCHE PATENTSCHRIFT**

(45) Veröffentlichungstag und Bekanntmachung des Hinweises auf die Patenterteilung:
**29.09.2021 Patentblatt 2021/39**

(51) Int Cl.:
*G01N 29/024* *(2006.01)*      *A61B 5/087* *(2006.01)*
*A61B 5/091* *(2006.01)*      *G01N 29/22* *(2006.01)*

(21) Anmeldenummer: **14198579.6**

(22) Anmeldetag: **17.12.2014**

(54) **Gerät für die Messung und Analyse des Multiple-Breath-Stickstoff-Auswaschverfahrens**

Device for measuring and analysing the multiple breath nitrogen washout method

Dispositif de mesure et d'analyse du procédé du lavage Multiple Breath à l'azote

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.04.2014 DE 102014004765**

(43) Veröffentlichungstag der Anmeldung:
**07.10.2015 Patentblatt 2015/41**

(73) Patentinhaber: **ndd Medizintechnik AG**
**8005 Zürich (CH)**

(72) Erfinder: **Buess, Christian, Dr.**
**8810 Horgen (CH)**

(74) Vertreter: **Maikowski & Ninnemann**
**Patentanwälte Partnerschaft mbB**
**Postfach 15 09 20**
**10671 Berlin (DE)**

(56) Entgegenhaltungen:
- **FLORIAN SINGER ET AL: "A Realistic Validation Study of a New Nitrogen Multiple-Breath Washout System", PLOS ONE, Bd. 7, Nr. 4, 27. April 2012 (2012-04-27), Seite e36083, XP055203322, DOI: 10.1371/journal.pone.0036083**
- **FLORIAN SINGER ET AL: "Tidal Volume Single Breath Washout of Two Tracer Gases - A Practical and Promising Lung Function Test", PLOS ONE, Bd. 6, Nr. 3, 10. März 2011 (2011-03-10), Seite e17588, XP055207522, DOI: 10.1371/journal.pone.0017588**
- **None**

EP 2 927 679 B1

**Beschreibung**

Die Erfindung betrifft ein Gerät für die Messung und Analyse des Multiple-Breath-Stickstoff-Auswaschverfahrens gemäß dem Oberbegriff des Anspruchs 1 sowie ein Verfahren zur Messung und Analyse eines Multiple-Breath-Auswaschvorgangs gemäß dem Oberbegriff des Anspruchs 9.

Die Messung des Auswaschvorgangs von Tracergasen aus der Lunge wird seit ca. 1950 durchgeführt und wurde seitdem genutzt, um Lungenvolumen, Unterteilungen des Lungenvolumens sowie Parameter der Ventilationsinhomogenität zu bestimmen [siehe: A.A. Hutchison, A.C. Sum, T.A. Demis, A. Erben, L.I. Landau. Momentanalyse eines Multiple-Breath Stickstoff Auswaschverfahrens bei Kindern. Am Rev Respir Dis 1982; 125: 28-32]. Das Multiple-Breath-Auswaschverfahren kann mit verschiedenen Tracergasen durchgeführt werden: Helium, SF6 (Schwefelhexafluorid) oder N2 (Stickstoff) sind die am häufigsten verwendeten Tracergase. Die Verwendung von N2 ist die einfachste Methode, da der Patient dabei nur von Luftatmung auf eine Atmung mit 100 % Sauerstoff umgestellt werden muss. Während der Sauerstoffatmung wird das in der Lunge verbleibende N2 Atemzug für Atemzug aus der Lunge ausgewaschen. Zur Messung der Konzentrationen der Tracergase N2, SF6 oder Helium wurden verschiedene Verfahren entwickelt, jedes mit individuellen Vor- und Nachteilen.

In der folgenden Beschreibung der bisher verwendeten Verfahren wird das auf Stickstoff (N2) basierende Auswaschverfahren beschrieben. Das Verfahren kann aber auf analoge Weise auch für die Analyse der Multiple-Breath-Auswaschung mit den Tracergasen SF6 oder Helium verwendet werden

Die Analyse mit dem Stickstoff-Multiple-Breath-Auswaschverfahren (MBW) benötigt eine präzise Messung der Flussgeschwindigkeit der Gase, welche in die Lunge hinein- und herausströmen, kombiniert mit einer ebenfalls präzisen Messung der N2-Konzentrationen, welche vom Patienten ein- und ausgeatmet werden. Sowohl Flussgeschwindigkeit als auch das N2-Signal müssen mit schnell reagierenden Sensoren gemessen werden, da selbst bei Ruheatmung die Fluss- und Konzentrationssignale schnellen Änderungen unterworfen sind. Zusätzlich müssen Fluss- und N2-Konzentrationssignal synchron (d.h. ohne zeitliche Verzögerung zwischen den Signalen) aufgezeichnet werden, da zur Bestimmung der ein- und ausgeatmeten N2-Volumen das Produkt von N2-Konzentration und Fluss integriert wird.

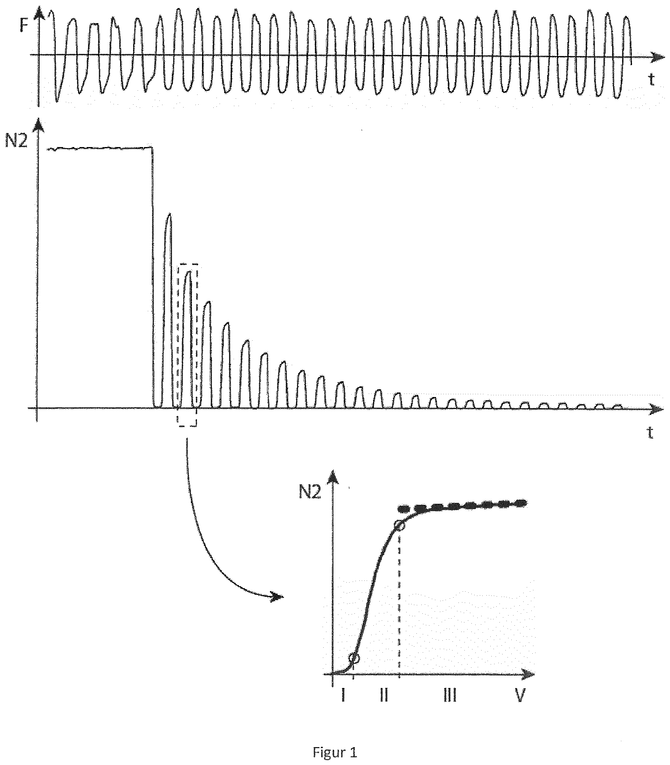

Figur 1 zeigt den Fluss (F) und die N2-Konzentration über die Zeit (t). Während des gesamten Tests atmet der Patient normal, aber mit aufgesetzter Nasenklammer, damit sämtliche Luft durch den Mund ein- und ausgeatmet wird. Vor dem eigentlichen N2-Auswaschvorgang ist die N2-Konzentration nahezu konstant und auf dem Wert der N2-Umgebungskonzentration (78,08 %); während des Auswaschvorgangs ist die N2-Konzentration während der Inspiration (nahe) Null, und während der Ausatmung nimmt die mittlere N2-Konzentration mit jedem Atemzug ab, da der Stickstoff mit jedem Atemzug weiter aus der Lunge ausgewaschen wird. Figur 1 enthält ebenfalls eine vergrößerte Darstellung der N2-Konzentration über das ausgeatmete Volumen (V). Diese Darstellung veranschaulicht die Phasen I bis III einer Ausatmung (I = Totraum, II = Mischluft, III = Alveolarluft). Durch die Bestimmung der Steigung der Phase III aller Atemzüge des Auswaschvorgangs können weitere Parameter zur Quantifizierung der Ventilationsinhomogenität bestimmt werden [Übereinkommenserklärung für Inertgas-Auswaschverfahrensmessungen bei Gebrauch von Multiple- und Single-Breath-Tests. Eur Respir J 2013; 41: 507-522].

Flussgeschwindigkeit und Gaskonzentrationssignale werden üblicherweise mit einer Messrate von 100 Hz erfasst. Strömungssensoren haben üblicherweise eine Ansprechzeit < 10 ms, die Sensoren zur Messung von Gaskonzentrationen sind meist langsamer mit Ansprechzeiten im Bereich von 60 bis 100 ms (siehe [Übereinkommenserklärung für Inertgas-Auswaschverfahrensmessungen bei Gebrauch von Multiple- und Single Breath Tests. Eur Respir J 2013; 41: 507-522]). Es stehen unterschiedliche Arten von Strömungssensoren für Gase zur Verfügung, wie z.B. Sensoren, die den Differenzdruck über einen Widerstand in der Strömungsstrecke erfassen, oder Sensoren, die das Abkühlen eines Hitzdrahtes messen, Sensoren, die die Rotation einer Turbine messen, oder Sensoren, die die Ultraschall-Laufzeit messen. Die Gaskonzentrationen unterliegen während des Auswaschvorgangs starken Schwankungen. Dies darf aber keinen Einfluss auf die Flussmessung haben oder dieser Einfluss muss ausreichend korrigiert werden. Doch nicht nur die Technik der Strömungssensoren ist unterschiedlich. Auch die Verfahren, wie die derzeitigen Systeme die Auswaschung durch Messung des Tracergases zur Bestimmung der N2-Konzentration analysieren, unterscheiden sich. Folgende Verfahren wurden zur Messung der N2-Konzentration eingesetzt:

1. Massenspektrometrie: Massenspektrometer können zur Messung der Gaskonzentrationen mit hoher Genauigkeit und schnellen Ansprechzeiten eingesetzt werden. Beim Multiple-Breath-Stickstoff-Auswaschverfahren werden üblicherweise die Werte der N2-, O2- und CO2-Konzentration gleichzeitig erfasst. Massenspektrometer kommen am häufigsten zum Einsatz, wenn für die Messung der Multiple-Breath-Auswaschung SF6 als Tracergas verwendet wird [P.M. Gustafsson, P. Aurora, A. Lindblad. Auswertung von fehlverteilter Beatmung als ein frühes Anzeichen für Lungenkrankheiten bei unter cystischen Fibrosen leidenden Kindern. Eur Respir J 2003; 22: 972-979]. Massenspektrometer sind sehr teuer und müssen regelmäßig gewartet werden.

2. Indirektes Verfahren: Durch die Verwendung zweier separater Gassensoren zur Messung der O2- und CO2-Konzentration kann die N2-Konzentration indirekt bestimmt werden. O2 wird gewöhnlich mit einem elektrochemischen Sensor oder mit Hilfe von Laserlicht durch IR-Absorption gemessen; für die CO2-Messung kommen üblicherweise IR-Absorptionssensoren zum Einsatz. Die N2-Konzentration wird indirekt anhand der O2- und CO2-Konzentration durch Anwendung des Daltonschen Gesetzes bestimmt, wonach die Summe aller Gase eines Gemischs 100% ergibt. Um eine präzise Bestimmung der N2-Konzentration sicherzustellen, müssen die O2- und CO2-Signale simultan gemessen werden, also ohne zeitliche Verzögerung zwischen den Signalen. Darüber hinaus müssen die Sensoren möglichst ähnliche Ansprechzeiten aufweisen [F. Singer, B. Houltz, P. Latzin, P. Robinson, P. Gustafsson. Eine realistische Validierungsstudie eines neuen Multiple-Breath-Stickstoff Auswaschverfahrens. PloS ONE 7(4): e36083, 2012].

F. Singer et al. Tidal volume single breath washout of two tracer gases-a practical and promising lung function test. PLoS One, 2011, 6. Jg., Nr. 3, S. e17588 beschreibt ein Atemanalysegerät mit zwei Ultraschall-Strömungs- und Molmassensensoren (einer im Hauptstrom, einer im Nebenstrom). Dabei wird nur der im Nebenstrom angeordnete Sensor dazu genutzt, die Molmasse der strömenden Gase zu bestimmen. Darüber hinaus wird gemäß der Lehre dieser Veröffentlichung mit relativen Konzentrationen gearbeitet. So wird dort insbesondere die Korrelation von Molmassenwerten, die mittels des Ultraschall-Strömungs- und Molmassensensors erhalten wurden, mit Molmassenwerten, die mittels eines Massenspektrometers erhalten wurden, beschrieben. Allerdings wird nicht die Möglichkeit vorgesehen, dass eine Hauptrecheneinheit dazu eingerichtet ist, die Stickstoffkonzentration in dem von dem Patienten ein- und ausgeatmeten Gas auf der Grundlage der Molmasse und der $CO_2$-Konzentration in dem von dem Patienten ein- und ausgeatmeten Gas zu berechnen.

Liegen die synchronen Signale der N2-Konzentration und des Gasflusses vor, wird das exspiratorische Stickstoff-Gesamtvolumen durch mathematische Integration der inspiratorischen und exspiratorischen N2-Konzentration multipliziert mit dem Fluss bestimmt. Es wird also das eingeatmete und ausgeatmete N2-Volumen jedes Atemzugs bestimmt und diese Volumina werden zur Ermittlung des gesamten ausgeatmeten N2-Volumens aufaddiert, bis ein Schwellwert für die N2-Konzentration erreicht wird (gewöhnlich <2,5% der Anfangskonzentration). Schließlich wird zur Berechnung der funktionellen Residualkapazität (FRC) das gesamte ausgeatmete N2-Volumen durch die Differenz aus der anfänglichen endtidalen N2-Konzentration und dem Endwert (Schwellwert) der endtidalen N2-Konzentration geteilt [Übereinkommenserklärung für Inertgas-Auswaschverfahrensmessungen bei Gebrauch von Multiple- und Single-Breath-Tests. Eur Respir J 2013; 41: 507-522]. Parameter der Ventilationsinhomogenität (wie z.B. LCI und Momente) werden durch eine detailliertere Analyse der Stickstoffkonzentration über die Zeit oder über das Volumen bestimmt.

Der Erfindung liegt die Aufgabe zugrunde, ein Gerät für das Messen und Analysieren von Multiple-Breath-Stickstoff-Auswaschverfahren bereitzustellen und ein gegenüber dem Stand der Technik verbessertes Verfahren zur Messung und Analyse eines Multiple-Breath-Auswaschvorgangs anzugeben.

Diese Aufgabe wird durch ein Gerät mit den Merkmalen des Anspruchs 1 und ein Verfahren mit den Merkmalen des Anspruchs 9 gelöst.

Bevorzugte Ausführungsformen der Erfindung gehen von den Unteransprüchen abhängend von Anspruch 1 hervor.

Die Erfindung verwendet die Fluss- und Molmassemessung mit Ultraschall [vergleiche EP 0597 060 B1, EP 0 653 919 B1] in Kombination mit einem schnell reagierenden CO2-Sensor, üblicherweise auf Basis des IR-Absorptionsverfahrens, zur Bestimmung der funktionellen Residualkapazität (FRC), des Lung Clearance Index (LCI), der Moment-Verhältnisse, der Phase-III-Analyse (einschliesslich Scond und Sacin) sowie weiterer, abgeleiteter Parameter eines Stickstoff-Multiple-Breath-Washout-Tests.

Vor der eigentlichen Auswaschphase, wenn der Patient Raumluft atmet, und während der Auswaschphase, wenn der Patient 100 % Sauerstoff atmet, müssen bei der Analyse folgende Gaskomponenten berücksichtigt werden:

- N2 (Stickstoff), in (trockener) Raumluft liegt die Konzentration bei etwa 78,08 %.

- 02 (Sauerstoff), in (trockener) Raumluft liegt die Konzentration bei etwa 20,95 %.

- CO2 (Kohlendioxid), in (trockener) Raumluft liegt die Konzentration bei 0,04 %, während der Ausatmung steigt sie auf etwa 4 bis 5 %.

- H2O (Wasserdampf), die Konzentration in Luft liegt, je nach Feuchtigkeit und Temperatur, zwischen 0 und etwa 5 %.

- Ar (Argon), in (trockener) Raumluft liegt die Konzentration bei etwa 0,93 %.

- Für alle anderen Gase (die sogenannten Spurengase) liegen die Konzentrationen unter 0,002 %. Diese Gase bleiben in der folgenden Diskussion unberücksichtigt.

Während des Multiple-Breath-Auswaschvorgangs schwankt die Konzentration sämtlicher Gase im Verlauf der In- und Exspiration. Unabhängig von diesen Änderungen in der Gaskonzentration, die hauptsächlich auf die Aufnahme von Sauerstoff und die Abgabe des entstandenen Kohlendioxids zurückzuführen sind, können die drei folgenden Gleichungen aufgestellt werden:

$$f_{N2} + f_{O2} + f_{CO2} + f_{H2O} + f_{Ar} = 100\% \tag{1}$$

$$f_{N2}M_{N2} + f_{O2}M_{O2} + f_{CO2}M_{CO2} + f_{H2O}M_{H2O} + f_{Ar}M_{Ar} = M \tag{2}$$

$$f_{Ar} = f_{N2} \cdot \frac{0.94}{78.2} \tag{3}$$

Hierbei ist $f_x$ der Anteil des Gases x und $M_x$ ist die Molmasse des Gases x in g/mol.

Gleichung (1) stellt dar, dass die Summe aller betrachteten Gasanteile 100 % ergibt (Daltonsches Gesetz). Wie oben erwähnt bleiben die Spurengase hierbei unberücksichtigt.

Gleichung (2) beschreibt die Messung der Molmasse: Die vom Ultraschallsensor gemessene Molmasse wird errechnet durch das Aufsummieren der Anteile jedes Gases und durch Multiplizieren mit der jeweiligen Molmasse.

In Gleichung (3) wird davon ausgegangen, dass der Anteil an Argon in einem festen Verhältnis zum Stickstoffanteil steht, dass also die Argon-Auswaschung direkt proportional zur Stickstoff-Auswaschung ist. Da keines dieser beiden Gase am Gasaustausch bei der Atmung eines Menschen beteiligt ist, ist dies eine valide Annahme.

Die folgende Liste gibt an, welche Variablen der Gleichungen (1) bis (3) gemessen werden und welche unbekannt sind:

Gemessene Variablen: fCO2, fH2O, M
Unbekannte Variablen: fN2, f02, fAr

Die jeweiligen Sensoren messen die CO2-Konzentrationen (fCO2) und die Molmasse (M). Die Wasserdampfkonzentration ($f_{H2O}$) in der Luft wird durch Messung der Feuchtigkeit, des Drucks und der Temperatur der Raumluft bestimmt. Aus diesen Werten kann die Konzentration des Wasserdampfs in der Raumluft ermittelt werden. Der mechanische Aufbau der Erfindung (siehe unten) gewährleistet während des gesamten Auswaschvorgangs eine definierte Wasserdampfkonzentration im Bereich des Sensors.

Da drei unbekannte Variablen und drei Gleichungen vorliegen, kann das System aufgelöst und die Konzentrationen für fN2, f02 und fAr können bestimmt werden. Daher lassen sich die Anteile fN2 und f02 aus den Messwerten für fCO2, fH2O und M bestimmen.

Die aufgeführten Gleichungen gelten für alle Phasen der Stickstoffauswaschung aus der Lunge eines Patienten; sie gelten ebenso für In-vitro-Systeme, also für Systeme zur Validierung medizinischer Geräte für die Analyse der Stickstoffauswaschung; zur Simulation der Patientenlunge können sich diese Systeme einer Vorrichtung wie einer Spritze und/oder eines Behälters bedienen [F. Singer, B. Houltz, P. Latzin, P. Robinson, P. Gustafsson. Eine realistische Validierungsstudie eines neuen Multiple-Breath-Stickstoff Auswaschverfahrens. PloS ONE 7(4): e36083, 2012].

Um eine bessere Genauigkeit zu erzielen, können die Werte für die Molmasse $M_x$ in Gleichung (2) durch korrigierte Molmassewerte $M^*_x$ ersetzt werden, wobei gilt $M^*_x = k_x * M_x$ und wobei es sich bei dem Faktor $k_x$ um eine dimensionslose Konstante für die adiabatische Indexkorrektur (also das Wärmekapazitätsverhältnis) handelt. Diese Korrektur ist deshalb erforderlich, weil die Messung der Molmasse (die gesamte Molmasse M in Gleichung (2)) auf einem festen Wärmekapazitätsverhältnis beruht (siehe [2]).

Um eine hinreichende Genauigkeit in der Beurteilung des Multiple-Breath-Auswaschvorgangs zur erzielen, müssen die Werte für fN2 und f02 mit relativ hoher Genauigkeit bestimmt werden (die Genauigkeit der N2-Konzentrationsmessung sollte <0,2 % sein [Übereinkommenserklärung für Inertgas-Auswaschverfahrenmessungen bei Gebrauch von Multiple- und Single Breath Tests. Eur Respir J 2013; 41: 507-522]). In diesem Zusammenhang sollte eine Querempfindlichkeit des CO2-Sensors gegen Sauerstoff berücksichtigt werden, wenn CO2 mit Hilfe der Infrarot-Absorption gemessen wird [R. Lauber, B. Seeberger, A.M. Zbinden. Kohlenstoffdioxid-Analsysatoren: Genauigkeit, Alarmgrenzwerte und Auswirkungen eingreifender Gase. Can J Anaesth 1995, 42:7, 643-656]. Diese zusätzliche Gleichung, die eine Korrektur des gemessenen CO2-Wertes mit der berechneten Sauerstoffkonzentration durchführt, kann einfach zu den Gleichungen (1) bis (3) hinzugefügt werden.

Das oben beschriebene Gerät nutzt gemäß der Erfindung eine Sensorentechnologie für das Messen von N2 Auswaschungen in einer neuen Art. Die Sensoren, die in dieser Ausführung genutzt werden, sind kostengünstig, haben eine hohe Langzeitstabilität, die es ermöglichen ein kosteneffektives Gerät für Multiple-Breath-Auswaschungs-Analysen zu bauen, die für eine Vielzahl von Patienten genutzt werden können (Neugeborene bis Erwachsene) und könnten auch an den Gebrauch bei ventilierten Patienten angepasst werden.

Weitere Merkmale, Details und Vorteile der Erfindung werden detaillierter mit Referenzen zu der Ausführungsform in der Zeichnung beschrieben.

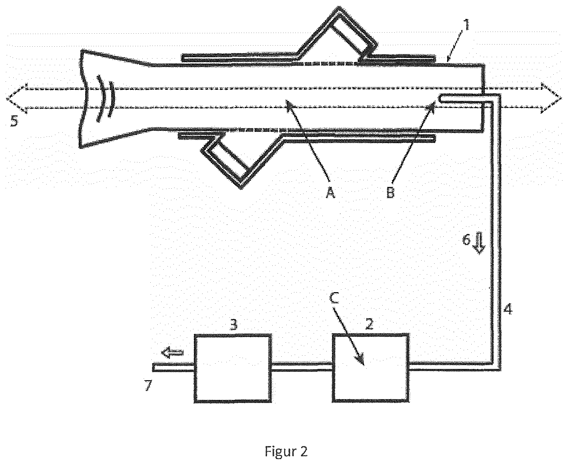

Für die Multiple-Breath-Auswaschanalyse nutzen die meisten Systeme eine Kombination aus Haupt- und Nebenstrommessung. Figur 2 erläutert dieses Konzept: Ein Strömungssensor mit Strömungsrohr (1) misst die Luft, die in die Lunge des Patienten und wieder herausströmt. Das strömende Gas wird in der Mitte des Strömungssensors (an der Stelle A) im Hauptstrom (5) gemessen. Da der Gassensor zu gross ist oder aufgrund von Einschränkungen im Messaufbau können Gaskonzentrationen in den meisten Fällen nicht direkt im Hauptstrom (5) gemessen werden. Ein geringer Anteil des Gases aus dem Hauptstrom wird daher über eine an der Stelle B angeordnete Probenspitze angesaugt. Mit Hilfe einer Pumpe (3) wird die Nebenstromprobe (6) über einen Schlauch dem Gassensor (2) zugeführt und anschliessend über den Auslass (7) abgeleitet. Der Probenschlauch (4) ist meist recht lang und hat einen kleinen Durchmesser (z.B. Länge von 1 m bei einem Durchmesser von 1 mm), so dass der Gassensor in einiger Entfernung zum Patienten platziert werden kann. Aufgrund der zeitlichen Verzögerung durch den Transport von Stelle B, an der die Nebenstromprobe in den Schlauch gelangt, bis Stelle C, wo die Gaskonzentration gemessen wird, sind die Gasflusssignale von Stelle A und C nicht mehr synchron. Für eine genaue Multiple-Breath-Auswaschanalyse ist die Synchronität jedoch eine Voraussetzung [Übereinkommenserklärung für Inertgas-Auswaschverfahrenmessungen bei Gebrauch von Multiple- und Single-Breath-Tests. Eur Respir J 2013; 41: 507-522]. Darüber hinaus existiert üblicherweise auch noch ein Abstand zwischen der Stelle A der Flussmessung und dem Punkt, an dem die Nebenstromprobe gezogen wird. Aufgrund dieses Abstands hängt die zeitliche Verzögerung zwischen Gasflusssignal und Gaskonzentrationssignal zusätzlich noch von der Geschwindigkeit und Richtung des Hauptstroms ab und nicht nur von der Geschwindigkeit der Nebenstromprobe. Mit der im Folgenden beschriebenen Ausgestaltung der Erfindung werden die beiden zeitlichen Verzögerungen kompensiert.

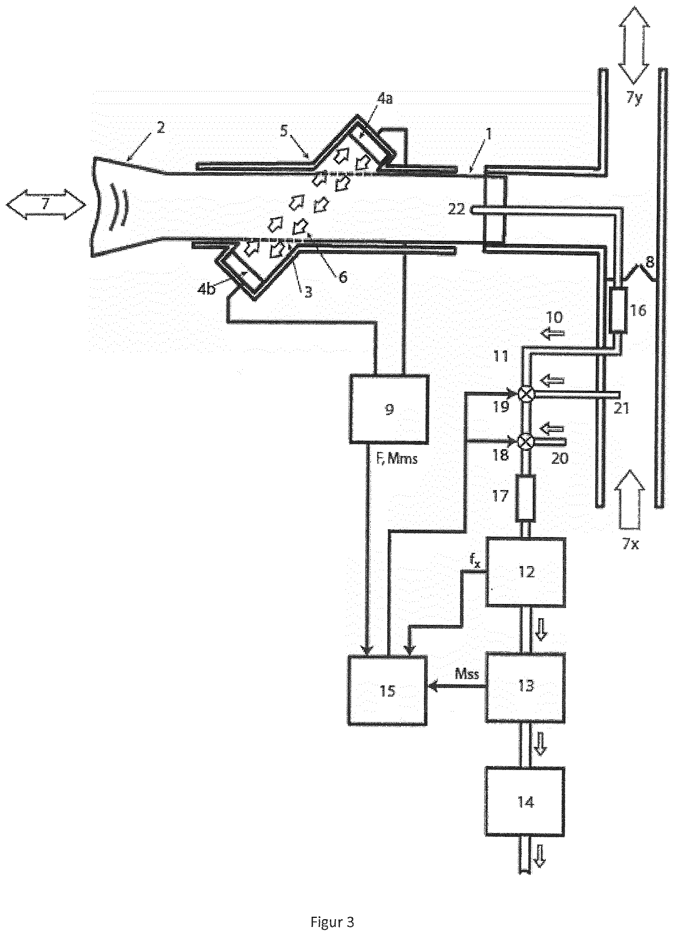

Figur 3 zeigt das Blockdiagramm eines Ausführungsbeispiels eines Systems für die Stickstoff-Multiple-Breath-Auswaschanalyse Der Patient atmet in einen Ultraschall-Strömungs- und Molmasse-Sensor (5). Die ein- und ausgeatmete Luft (7) strömt durch das Atemrohr (1) mit Mundstück (2), das ausgetauscht und entsorgt werden kann, um eine Kreuzkontamination zwischen Patienten zu vermeiden. Die Strömungsgeschwindigkeit der Luft sowie die Molmasse im Atemrohr werden mit Hilfe der Ultraschall-Transitzeitmessung (Laufzeitmessung) ermittelt: Zwei Ultraschall-Transducer (4a, 4b) senden Ultraschallimpulse (6) mit und entgegen dem Atemstrom im Atemrohr aus. Die Ultraschallimpulse passieren das feine Gewebe (3) im Atemrohr. Eine Elektronikeinheit (9) löst die Aussendung der Impulse aus, misst die Transitzeiten der Ultraschallimpulse stromauf- und -abwärts (4a) und (4b) und berechnet anhand dieser Transitzeiten die Strömungsgeschwindigkeit (F) sowie die Molmasse (Mms) im Hauptstrom [EP 0597 060 B1, EP 0 653 919 B1]. Die Ergebnisse für Strömung und Molmasse im Hauptstrom werden von der Elektronikeinheit(9) an die Hauptrecheneinheit (15) weitergeleitet. In der aktuellen Ausgestaltung liegt die Mess- und Übertragungsfrequenz dieser Signale zur Recheneinheit (15) bei 200 Hz.

Das Ende des Atemrohrs (1) ist in ein T-Rohr eingesteckt. In der Phase vor der eigentlichen Auswaschung atmet der Patient über einen Anschluss, der offen zur Raumluft ist (7y), Luft ein und aus. Ein Einwegventil (8) stellt sicher, dass kein Gas aus dem anderen Teil des T-Rohrs eingeatmet wird. Wird auf Sauerstoffatmung umgeschaltet, d.h. wenn die eigentliche Stickstoffauswaschung mit dem Multiple-Breath-Verfahren stattfindet, atmet der Patient Sauerstoff ein, der über den Anschluss (7x) mit konstanter oder variabler Geschwindigkeit zugeführt wird, und atmet über den Anschluss (7y) wieder aus. Der Fluss des am Anschluss (7x) zugeführten Sauerstoffs muss grösser sein als der maximale Inspirationsfluss des Patienten. In der Klinik kann Sauerstoff über den Wandanschluss zur Verfügung gestellt werden und ansonsten aus einer Sauerstoffflasche. Der Patient atmet daher nur während der Auswaschphase Sauerstoff aus dem Querstrom ein; der Patient atmet während der Auswaschphase des Tests keine Raumluft ein. Für die Gasanalyse wird eine Probe aus dem Nebenstrom (10) entnommen. Eine Pumpe (14) fördert die Gasprobe (10) durch einen Probenschlauch (11) zum CO2-Sensor (12) und zum Molmassesensor (13).

Für die Gasanalyse muss die Gasprobe aus dem Nebenstrom durch den Probenschlauch fliessen, um zu den Gasanalysatoren für die Messung der Molmasse (13) und des CO2 (12) zu gelangen. Die durch den Transport verursachte Verzögerung hängt von verschiedenen Variablen ab (z.B. vom Umgebungsdruck, von der Pumpgeschwindigkeit, usw.) und sie kann auch durch geringfügige Änderungen im Testaufbau von Test zu Test unterschiedlich sein. Wenn beispielsweise der verwendete Probenschlauch (11) ein austauschbares Teil ist, können Durchmesser und Länge von Test zu Test leicht variieren. Für die Multiple-Breath-Auswaschanalyse müssen das eingeatmete und das ausgeatmete N2-Volumen bestimmt werden. Dies geschieht durch Multiplikation der Stickstoffkonzentration mit der Flussgeschwindigkeit des Hauptstroms und anschliessender Integration über die Zeit. Die korrekte Funktionsweise dieses Verfahrens ist nur dann gewährleistet, wenn Fluss- und N2-Konzentrationssignal synchron sind; Zeitverzögerungen zwischen dem Signal der Flussgeschwindigkeit des Hauptstroms und den Gassignalen des Nebenstroms führen zu einer fehlerbehafteten Bestimmung der ein- und ausgeatmeten Gasvolumen [Übereinkommenserklärung für Inertgas-Auswaschverfahrenmessungen bei Gebrauch von Multiple- und Single-Breath-Tests. Eur Respir J 2013; 41: 507-522]. Das mit Sensor (13) gemessene Molmassesignal und das mit Sensor (12) gemessene CO2-Signal müssen daher mit dem Flussgeschwindigkeitssignal, das in der Mitte zwischen den beiden Ultraschall-Transducers (4a) und (4b) gemessen wird, synchronisiert werden. Die Synchronisation kann durch Kreuzkorrelation des Molmassesignals aus dem Hauptstrom (Mms) und dem Molmassesignal aus dem Nebenstrom (Mss) erreicht werden.Dieses Verfahren ist im Patent [EP 1 764 036 B1] beschrieben; eine Anwendungsmöglichkeit für die CO-Diffusionsmessung SB (single breath, Einatemzugmethode) ist im Patent [EP 1 764 035] beschrieben.

Die Recheneinheit (15) steuert zwei Ventile (18) und (19). Diese beiden Ventile dienen dazu, den Nebenstromfluss zwischen den Probenanschlüssen (22), (21) und (20) umzuschalten. Der Probenanschluss (22) ist der primäre Probenanschluss, der während des Tests genutzt wird. Der Probenanschluss (21) wird nur während der Kalibrationsphase des Tests genutzt. Über diesen Anschluss werden Proben des Gases gezogen, das dem Patienten während der Auswaschphase (100 % Sauerstoff) zugeführt wird. Dies ist ein erster Kalibrationspunkt für den Molmassesensor (13). Auch der Probenanschluss (20) wird nur während der Kalibrationsphase des Tests genutzt. Über diesen Anschluss werden Proben der Raumluft gezogen. Dieses Gas dient als zweiter Kalibrationspunkt für den Molmasse-sensor (13) und als Nullreferenz für den CO2-Sensor (12).

Weitere Elemente im Nebenstrom dienen der Aufbereitung des Gases, bevor es in die Gassensoren gelangt. Schlauchmaterial, das für Wasserdampf durchlässig ist (16), z.B. mit Nafion®-Membran, wird zur Reduzierung der Feuchtigkeit (Wasserdampf) des Nebenstromgases verwendet. Der Schlauch (16) wird üblicherweise in der Nähe des Probenanschlusses 22 im Bereich der Sauerstoffzufuhr platziert, denn dies reduziert das Risiko einer Wasserkondensation im Nebenstrombereich des Systems, da die vom Patienten ausgeatmete Luft mit 100 % Wasserdampf gesättigt ist. Die Platzierung innerhalb der Sauerstoffzufuhr reduziert die Wasserdampfmenge ganz wesentlich, da der eingeleitete Sauerstoff frei von Wasserdampf ist. Weiteres Schlauchmaterial, das für Wasserdampf durchlässig ist (17) kann eingesetzt werden, damit der Nebenstromfluss bei Eintritt in die Gassensoren einen definierten Wasserdampf-Partialdruck aufweist. Dieses zusätzliche, für Wasserdampf durchlässige Schlauchmaterial, gewährleistet einen einheitlichen Wasserdampf-Partialdruck aller Messgasproben des Nebenstroms von den Anschlüssen 20, 21 und 22, und zwar unabhängig davon, dass das Messgas der einen Quelle wasserdampffrei ist (Gas von Anschluss 21), das Messgas der zweiten Quelle die Feuchtigkeit der Raumluft aufweist (Anschluss 20) und der Wasserdampfgehalt der dritte Quelle schwankt (Anschluss 22).

Die Neuerung des beschriebenen Analysesystems für den Multiple-Breath-Auswaschvorgang besteht in der Berechnung der Stickstoffkonzentration auf der Basis der Signale eines Molmassesensors und eines herkömmlichen Infrarot-CO2-Sensors. Eine weitere Neuerung ist die automatische Kalibrierung der Gassensoren mit Raumluft und mit dem Auswaschgas (Sauerstoff). In ähnlicher Weise kann das System aufgebaut werden, das die Stickstoffkonzentration auf der Basis der Signale eine Molmassesensors und eines herkömmlichen O2-Sensors berechnet.

## Patentansprüche

1. Gerät zur Messung und Analyse des Multiple-Breath-Auswaschvorgangs, mit einem Strömungsrohr (1), durch das Gas von und zu der Lunge eines Patienten strömen kann und in dessen Mitte strömendes Gas in einem Hauptstrom (5) gemessen werden kann,

einem ersten Ultraschall-Strömungs- und Molmassesensor (4a, 4b), der dazu dient, den momentanen Fluss und die momentane Molmasse eines von einem Patienten ein- und ausgeatmeten Gases (7) in dem Hauptstrom (5) zu messen,
einem $CO_2$-Sensor (12) in einem vom Hauptstrom (5) räumlich getrennten Nebenstrom (10), welcher einen kleinen, aus dem Hauptstrom (5) entnommenen Gasanteil darstellt, wobei der $CO_2$-Sensor (12) dazu dient, die $CO_2$-Konzentration in dem von dem Patienten ein- und ausgeatmeten Gas (7) zu messen,
einem zweiten Ultraschall-Strömungs- und Molmassesensor (13) im Nebenstrom (10), der dazu dient, die Molmasse des von dem Patienten ein- und ausgeatmeten Gases (7) im Nebenstrom (10) zu messen,
einer mit dem ersten Ultraschall-Strömungs- und Molmassesensor (4a, 4b) verbundenen Elektronikeinheit (9), die dazu dient, den Fluss und die Molmasse des von dem Patienten ein- und ausgeatmeten Gases (7) zu berechnen, und
einer mit der Elektronikeinheit (9), dem $CO_2$-Sensor (12) und dem zweiten Ultraschall-Strömungs- und Molmassesensor (13) verbundenen Hauptrecheneinheit (15),
wobei die Hauptrecheneinheit (15) dazu dient, die $N_2$-Konzentration in dem von dem Patienten ein- und ausgeatmeten Gas (7) auf der Grundlage der Molmasse des von dem Patienten ein- und ausgeatmeten Gases (7), der $CO_2$-Konzentration in dem von dem Patienten ein- und ausgeatmeten Gas (7) und eines vorbestimmten Werts für die Wasserdampfkonzentration durch Lösung der folgenden drei Gleichungen zu bestimmen:

$$f_{N2} + f_{O2} + f_{CO2} + f_{H2O} + f_{Ar} = 100\ \% \qquad\qquad (1)$$

$$f_{N2}M_{N2} + f_{O2}M_{O2} + f_{CO2}M_{CO2} + f_{H2O}M_{H2O} + f_{Ar}M_{Ar} = M \qquad\qquad (2)$$

$$f_{Ar} = f_{N2} \cdot \frac{0{,}94}{78{,}2} \qquad\qquad (3)$$

wobei $f_x$ der Anteil des Gases x und $M_x$ die Molmasse des Gases x in g/mol ist,
wobei die mit diesen Gleichungen berechnete $N_2$-Konzentration und der gemessene Fluss des Hauptstroms für jeden Atemzug zur Bestimmung folgender Werte verwendet werden:

a) des gesamten inspiratorischen und exspiratorischen Gasvolumens des Patienten durch mathematische Integration des Flusses,
b) des inspiratorischen und exspiratorischen $N_2$-Gasvolumens des Patienten durch mathematische Integration des Produkts aus inspiratorischer bzw. exspiratorischer $N_2$-Konzentration und dem Fluss und
c) von Parametern, die aus dem inspiratorischen und exspiratorischen $N_2$-Gasvolumen und/oder dem gesamten inspiratorischen und exspiratorischen Gasvolumen abgeleitet sind und ausgewählt sind aus der Gruppe bestehend aus der funktionellen Residualkapazität, dem Lung-Clearance-Index, Momenten und Momentverhältnissen, der Phase-III-Steigung und den daraus abgeleiteten Parametern $S_{cond}$ und $S_{acin}$, wobei

i. zur Berechnung der funktionellen Residualkapazität das gesamte ausgeatmete $N_2$-Volumen durch die Differenz aus der anfänglichen endtidalen $N_2$-Konzentration und dem Endwert der endtidalen $N_2$-Konzentration geteilt wird,
ii. der Lung-Clearance-Index die Anzahl der Lungendurchsätze, berechnet aus dem gesamten kumulativen exspiratorischen Gasvolumen dividiert durch die funktionelle Residualkapazität, angibt, die erforderlich sind, um die $N_2$-Gaskonzentration auf einen bestimmten Bruchteil ihrer Ausgangskonzentration zu reduzieren,
iii. die Momente das nullte Moment, das erste Moment und das zweite Moment sind, wobei das nullte Moment bestimmt wird durch Berechnung der Fläche unter einer Kurve der endtidalen Konzentration des Stickstoffs aufgetragen über die Anzahl der Lungendurchsätze, wobei das erste Moment bestimmt wird durch Berechnung der Fläche unter einer Kurve der endtidalen Konzentration des Stickstoffs multipliziert mit der Anzahl der Lungendurchsätze aufgetragen über die Anzahl der Lungenvolumendurchsätze und wobei das zweite Moment bestimmt wird durch Berechnung der Fläche unter einer Kurve der endtidalen Konzentration des Stickstoffs multipliziert mit dem Quadrat der Anzahl der Lungenvolumendurchsätze aufgetragen über die Anzahl der Lungenvolumendurchsätze,
iv. die Momentverhältnisse das Verhältnis des ersten Moments zum nullten Moment sowie das Verhältnis des zweiten Moments zum nullten Moment umfassen,
v. die Phase-III-Steigung die Steigung einer Kurve der $N_2$-Konzentration aufgetragen über das gesamte exspiratorische Gasvolumen in einem Bereich von 25 % bis 75 % des gesamten exspiratorischen Gasvolumens ist,
vi. $S_{cond}$ als lineare Regression der Phase-III-Steigungen zwischen etwa 1,5 und 6,0 Lungendurchsätzen berechnet wird,
vii. $S_{acin}$ drei technisch akzeptable Werte der Phase-III-Steigung für den ersten Atemzug erfordert und als der mittlere Wert der Phase-III-Steigung der drei ersten Atemzüge abzüglich des $S_{cond}$-Beitrags, basierend auf dem mittleren Lungendurchsatzwert der drei ersten Atemzüge, berechnet wird.

2. Gerät nach Anspruch 1, **dadurch gekennzeichnet, dass** die Molmassewerte der einzelnen Gase $M_x$ ersetzt werden durch einen Molmassewert $M^*_x = k_x * M_x$, wobei es sich bei $k_x$ um eine dimensionslose Konstante für das Gas x zur adiabatischen Indexkorrektur handelt.

3. Gerät nach Anspruch 1 oder 2, **dadurch gekennzeichnet, dass** eine $O_2$-Querempfindlichkeit des $CO_2$-Sensors kompensiert wird, indem die Gleichungen um eine geeignete Korrekturgleichung ergänzt werden und die Gleichungen nach $N_2$ aufgelöst werden.

4. Gerät nach einem der vorherigen Ansprüche, **dadurch gekennzeichnet, dass** die Hauptrecheneinheit (15) auch dazu dient, eine durch einen Transport des von dem Patienten ein- und ausgeatmeten Gases (7) aus dem Hauptstrom (1) in den Nebenstrom (10) verursachte zeitliche Verzögerung zwischen der Flussmessung im Hauptstrom (1) und der Messung der $CO_2$-Konzentration und der Molmasse im Nebenstrom (10) durch eine Bestimmung der Verzögerung anhand einer mathematischen Kreuzkorrelation der Molmassemessung im Hauptstrom (1) und im Nebenstrom (10) zu korrigieren.

5. Gerät nach einem der vorherigen Ansprüche, **dadurch gekennzeichnet, dass** die Hauptrecheneinheit (15) auch dazu dient, eine zusätzliche Zeitverzögerung aufgrund eines Abstands zwischen der Mitte der Flussmessung im Hauptstrom (1) und einem Punkt zur Probenentnahme (22) für den Nebenstrom (10) durch Berücksichtigung einer Verzögerungszeit zu kompensieren, wobei diese Verzögerungszeit mit einer Funktion auf Basis der Flussgeschwindigkeit des Hauptstroms (1) und der Flussrichtung berechnet wird.

6. Gerät nach einem der vorherigen Ansprüche, **dadurch gekennzeichnet, dass** die Hauptrecheneinheit (15) auch dazu dient, unterschiedliche Ansprechzeiten des $CO_2$-Sensors (12) sowie des ersten Ultraschall-Strömungs- und Molmassesensors (4a, 4b) und/oder des zweiten Ultraschall-Strömungs- und Molmassesensors (13) durch mathematisches Verlangsamen oder Beschleunigen der Geschwindigkeit des $CO_2$-Sensors (12) bzw. des ersten Ultraschall-Strömungs- und Molmassesensors (4a, 4b) und/oder des zweiten Ultraschall-Strömungs- und Molmassesensors (13) zu kompensieren, um eine bei den Sensoren (12, 4a, 4b, 13) (nahezu) gleiche Ansprechzeit zu erzielen.

7. Gerät nach einem der vorherigen Ansprüche, **dadurch gekennzeichnet, dass** die Hauptrecheneinheit (15) auch dazu dient, einen Offset des $CO_2$-Sensors (12) automatisch mit einer der Raumluft entnommenen Gasprobe zu kalibrieren.

8. Gerät nach einem der vorherigen Ansprüche, **dadurch gekennzeichnet, dass** die Hauptrecheneinheit (15) auch dazu dient, einen Offset und eine Verstärkung des zweiten Ultraschall-Strömungs- und Molmassesensors (13) automatisch mit Gasproben aus der Raumluft und aus reinem Sauerstoff, welcher als Auswaschgas verwendet wird, zu kalibrieren.

9. Verfahren zur Messung und Analyse eines Multiple-Breath-Auswaschvorgangs unter Verwendung eines Geräts nach einem der vorhergehenden Ansprüche, umfassend die folgenden Schritte:

   • Messen des momentanen Flusses und der momentanen Molmasse eines von einem Patienten ein- und ausgeatmeten Gases (7) in einem Hauptstrom (1),
   • Messen einer $CO_2$-Konzentration in dem von dem Patienten ein- und ausgeatmeten Gas (7) in einem Nebenstrom (10),
   • Messen der Molmasse des von dem Patienten ein- und ausgeatmeten Gases (7) in dem Nebenstrom (10),
   • Berechnen der $N_2$-Konzentration in dem von dem Patienten ein- und ausgeatmeten Gas (7) auf der Grundlage der Molmasse des von dem Patienten ein- und ausgeatmeten Gases (7), der $CO_2$-Konzentration in dem von dem Patienten ein- und ausgeatmeten Gas (7) und eines vorbestimmten Werts für die Wasserdampfkonzentration durch Lösung der folgenden drei Gleichungen:

$$f_{N2} + f_{O2} + f_{CO2} + f_{H2O} + f_{Ar} = 100\,\% \tag{1}$$

$$f_{N2}M_{N2} + f_{O2}M_{O2} + f_{CO2}M_{CO2} + f_{H2O}M_{H2O} + f_{Ar}M_{Ar} = M \tag{2}$$

$$f_{Ar} = f_{N2} \cdot \frac{0,94}{78,2} \tag{3}$$

wobei $f_x$ der Anteil des Gases x und $M_x$ die Molmasse des Gases x in g/mol ist.

10. Verfahren nach Anspruch 9, **dadurch gekennzeichnet, dass** die $N_2$-Konzentration in dem von dem Patienten ein- und ausgeatmeten Gas (7) mit dem gemessenen Fluss multipliziert wird und das derart erhaltene eingeatmete $N_2$-Volumen und ausgeatmete $N_2$-Volumen jedes Atemzugs zu einem gesamten ausgeatmeten $N_2$-Volumen addiert werden, bis ein Schwellenwert für die $N_2$-Konzentration erreicht wird.

**Claims**

1. A device for measuring and analyzing the multiple-breath washout process, comprising

a flow tube (1) through which gas can flow to and from the lungs of a patient, and gas flowing in its middle can be measured in a main stream (5),

a first ultrasonic flow and molar mass sensor (4a, 4b), which serves to measure the current flux and the current molar mass of a gas (7) inhaled and exhaled by a patient in the main stream (5),

a $CO_2$ sensor (12) in a side stream (10) spatially separated from the main stream (5), which represents a small gas fraction taken from the main stream (5), wherein the $CO_2$ sensor (12) serves to measure the $CO_2$ concentration in the gas (7) inhaled and exhaled by the patient,

a second ultrasonic flow and molar mass sensor (13) in the side stream (10), which serves to measure the molar mass of the gas (7) inhaled and exhaled by the patient in the side stream (10),

an electronic unit (9) connected to the first ultrasonic flow and molar mass sensor (4a, 4b), which serves to calculate the flux and the molar mass of the gas (7) inhaled and exhaled by the patient, and

a main computing unit (15) connected to the electronic unit (9), the $CO_2$ sensor (12) and the second ultrasonic flow and molar mass sensor (13),

wherein the main computing unit (15) serves to determine the $N_2$ concentration in the gas (7) inhaled and exhaled by the patient on the basis of the molar mass of the gas (7) inhaled and exhaled by the patient, the $CO_2$ concentration in the gas (7) inhaled and exhaled by the patient, and a predetermined value for the steam concentration by solving the following three equations:

$$f_{N2} + f_{O2} + f_{CO2} + f_{H2O} + f_{Ar} = 100\,\% \tag{1}$$

$$f_{N2}M_{N2} + f_{O2}M_{O2} + f_{CO2}M_{CO2} + f_{H2O}M_{H2O} + f_{Ar}M_{Ar} = M \tag{2}$$

$$f_{Ar} = f_{N2} \cdot \frac{0,94}{78,2} \tag{3}$$

wherein $f_x$ is the fraction of the gas x and $M_x$ is the molar mass of the gas x in g/mol,

wherein the $N_2$ concentration calculated by means of these equations and the measured flux of the main stream for each breath are used to determine the following values:

a) of the total inspiratory and expiratory gas volume of the patient by mathematical integration of the flux,

b) of the inspiratory and expiratory $N_2$ gas volume of the patient by mathematical integration of the product from the inspiratory or expiratory $N_2$ concentration and the flux, and

c) of parameters derived from the inspiratory and expiratory $N_2$ gas volume and/or the total inspiratory and expiratory gas volume and selected from the group consisting of the functional residual capacity, the lung clearance index, moments and moment ratios, the phase-III slope and the derived parameters $S_{cond}$ and $S_{acin}$, wherein

i. for calculating the functional residual capacity, the total exhaled $N_2$ volume is divided by the difference between the initial end-tidal $N_2$ concentration and the final value of the end-tidal $N_2$ concentration,

ii the lung clearance index indicates the number of lung throughputs, calculated from the total cumulative expiratory gas volume divided by the functional residual capacity, lung throughputs which are required to reduce the $N_2$ gas concentration to a certain fraction of its initial concentration,

iii. the moments are the zeroth moment, the first moment and the second moment, wherein the zeroth moment is determined by calculating the area under a curve of the end-tidal concentration of the nitrogen, plotted over the number of lung throughputs, wherein the first moment is determined by calculating the area under a curve of the end-tidal concentration of the nitrogen multiplied by the number of lung throughputs plotted over the number of lung volume throughputs, and wherein the second moment is determined by calculating the area under a curve of the end-tidal concentration of the nitrogen multiplied by the square of the number of lung volume throughputs plotted over the number of lung volume throughputs,

iv. the moment ratios comprise the ratio of the first moment to the zeroth moment and the ratio of the second moment to the zeroth moment,

v. the phase-III slope is the slope of a curve of the $N_2$ concentration plotted over the total expiratory gas volume in a range from 25% to 75% of the total expiratory gas volume,

vi. $S_{cond}$ is calculated as a linear regression of phase III slopes between about 1.5 and 6.0 lung throughputs,

vii. $S_{acin}$ requires three technically acceptable values of the phase-III slope for the first breath and is calculated as the mean value of the phase-III slope of the first three breaths minus the $S_{cond}$ contribution, based on the mean lung throughput value of the first three breaths.

2. The device according to claim 1, **characterized in that** the molar mass values of the individual gases $M_x$ are replaced by a molar mass value $M^*_x = k_x * M_x$, wherein $k_x$ is a dimensionless constant for the gas x for adiabatic index correction.

3. The device according to claim 1 or 2, **characterized in that** an $O_2$ cross-sensitivity of the $CO_2$ sensor is compensated by supplementing the equations with a suitable correction equation and by solving the equations for $N_2$.

4. The device according to any of the preceding claims, **characterized in that** the main computing unit (15) also serves to correct a time delay caused by a transport of the gas (7) inhaled and exhaled by the patient from the main stream (1) into the side stream (10) between the flux measurement in the main stream (1) and the measurement of the $CO_2$ concentration and the molar mass in the side stream (10) by determining the delay using a mathematical cross correlation of the molar mass measurement in the main stream (1) and in the side stream (10).

5. The device according to any of the preceding claims, **characterized in that** the main computing unit (15) also serves to compensate an additional time delay due to a distance between the center of the flux measurement in the main stream (1) and a point for sampling (22) for the side stream (10) by taking account of a delay time, wherein this delay time is calculated by using a function based on the flow velocity of the main stream (1) and the flow direction.

6. The device according to any of the preceding claims, **characterized in that** the main computing unit (15) also serves to compensate different response times of the $CO_2$ sensor (12) and of the first ultrasonic flow and molar mass sensor (4a, 4b) and/or of the second ultrasonic flow and molar mass sensor (13) by mathematically slowing down or accelerating the speed of the $CO_2$ sensor (12) or of the first ultrasonic flow and molar mass sensor (4a, 4b) and/or of the second ultrasonic flow and molar mass sensor (13) in order to achieve an (almost) identical response time for the sensors (12, 4a, 4b, 13).

7. The device according to any of the preceding claims, **characterized in that** the main computing unit (15) also serves to automatically calibrate an offset of the $CO_2$ sensor (12) with a gas sample taken from the room air.

8. The device according to any of the preceding claims, **characterized in that** the main computing unit (15) also serves to automatically calibrate an offset and an amplification of the second ultrasonic flow and molar mass sensor (13) with gas samples from the room air and from pure oxygen, which is used as a washout gas.

9. A method for measuring and analyzing a multiple-breath washout process by using a device according to any of the preceding claims, comprising the following steps:

   • measuring the current flux and the current molar mass of a gas (7) inhaled and exhaled by a patient in a main stream (1),
   • measuring a $CO_2$ concentration in the gas (7) inhaled and exhaled by the patient in a side stream (10),
   • measuring the molar mass of the gas (7) inhaled and exhaled by the patient in the side stream (10),
   • calculating the $N_2$ concentration in the gas (7) inhaled and exhaled by the patient on the basis of the molar mass of the gas (7) inhaled and exhaled by the patient, the $CO_2$ concentration in the gas (7) inhaled and exhaled by the patient, and a predetermined value for the steam concentration by solving the following three equations:

$$f_{N2} + f_{O2} + f_{CO2} + f_{H2O} + f_{Ar} = 100\,\% \tag{1}$$

$$f_{N2}M_{N2} + f_{O2}M_{O2} + f_{CO2}M_{CO2} + f_{H2O}M_{H2O} + f_{Ar}M_{Ar} = M \tag{2}$$

$$f_{Ar} = f_{N2} \cdot \frac{0,94}{78,2} \qquad (3)$$

wherein $f_x$ is the fraction of the gas x, and $M_x$ is the molar mass of the gas x in g/mol.

**10.** The method according to claim 9, **characterized in that** the $N_2$ concentration in the gas (7) inhaled and exhaled by the patient is multiplied by the measured flux, and the $N_2$ volume inhaled and the $N_2$ volume exhaled with each breath, which are obtained in this way, are added to a total $N_2$ volume exhaled, until a threshold value for the $N_2$ concentration is reached.

**Revendications**

**1.** Dispositif de mesure et d'analyse du processus de lavage Multiple Breath, comportant

un tube d'écoulement (1), par lequel du gaz peut circuler à partir et vers les poumons d'un patient, et du gaz circulant au centre duquel peut être mesuré dans un courant principal (5),
un premier capteur d'écoulement et de masse molaire à ultrasons (4a, 4b), qui sert à mesurer le flux instantané et la masse molaire instantanée d'un gaz (7) inspiré et expiré par un patient dans le courant principal (5),
un capteur de $CO_2$ (12) dans un courant secondaire (10) séparé spatialement du courant principal (5), qui représente une petite fraction de gaz prélevée du courant principal (5), le capteur de $CO_2$ (12) servant à mesurer la concentration en $CO_2$ dans le gaz (7) inspiré et expiré par le patient,
un deuxième capteur d'écoulement et de masse molaire à ultrasons (13) dans le courant secondaire (10), qui sert à mesurer la masse molaire du gaz (7) inspiré et expiré par le patient dans le courant secondaire (10),
une unité électronique (9) connectée au premier capteur d'écoulement et de masse molaire à ultrasons (4a, 4b), qui sert à calculer le flux et la masse molaire du gaz (7) inspiré et expiré par le patient, et
une unité de calcul principale (15) connectée à l'unité électronique (9), au capteur de $CO_2$ (12) et au deuxième capteur d'écoulement et de masse molaire à ultrasons (13), dans lequel l'unité de calcul principale (15) sert à déterminer la concentration en $N_2$ dans le gaz (7) inspiré et expiré par le patient sur la base de la masse molaire du gaz (7) inspiré et expiré par le patient, la concentration en $CO_2$ dans le gaz (7) inspiré et expiré par le patient, et une valeur prédéterminée pour la concentration en vapeur d'eau par en résolvant les trois équations suivantes :

$$f_{N2} + f_{O2} + f_{CO2} + f_{H2O} + f_{Ar} = 100\,\% \qquad (1)$$

$$f_{N2}M_{N2} + f_{O2}M_{O2} + f_{CO2}M_{CO2} + f_{H2O}M_{H2O} + f_{Ar}M_{Ar} = M \qquad (2)$$

$$f_{Ar} = f_{N2} \cdot \frac{0,94}{78,2} \qquad (3)$$

dans lequel $f_x$ représente la proportion de gaz x et $M_x$ représente la masse molaire du gaz x en g/mol,
dans lequel la concentration en $N_2$ calculée à l'aide de ces équations et le flux mesuré du courant principal pour chaque respiration sont utilisés pour déterminer les valeurs suivantes :

a) du volume total de gaz inspiratoire et expiratoire du patient par intégration mathématique du flux,
b) du volume de gaz $N_2$ inspiratoire et expiratoire du patient par intégration mathématique du produit de la concentration en $N_2$ inspiratoire ou expiratoire et du flux, et
c) de paramètres dérivés du volume de gaz N2 inspiratoire et expiratoire et/ou du volume total de gaz inspiratoire et expiratoire et sélectionnés à partir du groupe constitué de la capacité résiduelle fonctionnelle, de l'indice de clairance pulmonaire, des moments et des rapports de moments, la pente de phase III et les paramètres qui en découlent $S_{cond}$ et $S_{acin}$, dans lequel

i. pour calculer la capacité résiduelle fonctionnelle, le volume total de N2 expiré est divisé par la différence entre la concentration initiale en $N_2$ en fin d'expiration et la valeur finale de la concentration en $N_2$ en fin d'expiration,

ii l'indice de clairance pulmonaire indique le nombre de débits pulmonaires, calculé à partir du volume total cumulé de gaz expiratoire divisé par la capacité résiduelle fonctionnelle, qui sont nécessaires pour réduire la concentration en gaz $N_2$ à une fraction déterminée de sa concentration initiale, iii. les moments sont le moment zéro, le premier moment et le deuxième moment, le moment zéro étant déterminé par calcul de la surface sous une courbe de la concentration en azote en fin d'expiration tracée sur le nombre de débits pulmonaires, dans lequel le premier moment est déterminé par calcul de la surface sous une courbe de la concentration en azote en fin d'expiration multipliée par le nombre de débits pulmonaires tracé sur le nombre de débits du volume pulmonaire et dans lequel le deuxième moment est déterminé par calcul de la surface sous une courbe de la concentration en azote en fin d'expiration multipliée par le carré du nombre de débits du volume pulmonaire tracé sur le nombre de débits du volume pulmonaire,

iv. les rapports de moment comprennent le rapport du premier moment par rapport au moment zéro, ainsi que le rapport du deuxième moment par rapport au moment zéro,

v. la pente de phase III est la pente d'une courbe de la concentration en N2 tracée sur tout le volume de gaz expiratoire dans une plage de 25 % à 75 % du volume total de gaz expiratoire,

vi. $S_{cond}$ est calculé comme régression linéaire des pentes de phase III entre environ 1,5 et 6,0 débits pulmonaires environ,

vii. $S_{acin}$ requiert trois valeurs techniquement acceptables de la pente de phase III pour la première respiration et est calculée comme la valeur moyenne de la pente de phase III des trois premières respirations moins la contribution $S_{cond}$, basée sur la valeur moyenne du débit pulmonaire des trois premières respirations.

2. Dispositif selon la revendication 1, **caractérisé en ce que** les valeurs de masse molaire des divers gaz $M_x$ sont remplacées par une valeur de masse molaire $M^*_x = k_x * M_x$, dans lequel $k_x$ est une constante sans dimension pour le gaz x pour la correction de l'indice adiabatique.

3. Dispositif selon la revendication 1 ou 2, **caractérisé en ce qu'**une sensibilité croisée à l'$O_2$ du capteur de $CO_2$ est compensée en ajoutant une équation de correction appropriée aux équations et en résolvant les équations en $N_2$.

4. Dispositif selon l'une quelconque des revendications précédentes, **caractérisé en ce que** l'unité de calcul principale (15) sert également à corriger un retard temporel causé par un transport du gaz (7) inspiré et expiré par le patient depuis le courant principal (1) dans le courant secondaire (10) entre la mesure du flux dans le courant principal (1) et la mesure de la concentration en $CO_2$ et de la masse molaire dans le courant secondaire (10) en déterminant le retard sur la base d'une corrélation croisée mathématique de la mesure de la masse molaire dans le courant principal (1) et dans le courant secondaire (10).

5. Dispositif selon l'une quelconque des revendications précédentes, **caractérisé en ce que** l'unité de calcul principale (15) sert également à compenser un retard temporel supplémentaire dû à une distance entre le centre de la mesure du flux dans le courant principal (1) et un point d'échantillonnage (22) pour le courant secondaire (10) en tenant compte d'un temps de retard, ledit temps de retard étant calculé avec une fonction basée sur la vitesse d'écoulement du courant principal (1) et la direction d'écoulement.

6. Dispositif selon l'une quelconque des revendications précédentes, **caractérisé en ce que** l'unité de calcul principale (15) sert également à compenser les différents temps de réponse du capteur de $CO_2$ (12) ainsi que du premier capteur d'écoulement et de masse molaire à ultrasons (4a, 4b) et/ou du deuxième capteur d'écoulement et de masse molaire à ultrasons (13) en ralentissant ou en accélérant mathématiquement la vitesse du capteur de $CO_2$ (12) ou du premier capteur d'écoulement et de masse molaire à ultrasons (4a, 4b) et/ou du deuxième capteur d'écoulement et de masse molaire à ultrasons (13), afin d'obtenir un temps de réponse (pratiquement) identique pour les capteurs (12, 4a, 4b, 13).

7. Dispositif selon l'une quelconque des revendications précédentes, **caractérisé en ce que** l'unité de calcul principale (15) sert également à calibrer automatiquement un décalage du capteur de $CO_2$ (12) avec un échantillon de gaz prélevé dans l'air ambiant.

8. Dispositif selon l'une quelconque des revendications précédentes, **caractérisé en ce que** l'unité de calcul principale (15) sert également à calibrer automatiquement un décalage et une amplification du deuxième capteur d'écoulement et de masse molaire à ultrasons (13) avec des échantillons de gaz provenant de l'air ambiant et de l'oxygène pur, qui est utilisé comme gaz de lavage.

9. Procédé de mesure et d'analyse d'un processus de lavage Multiple Breath en utilisant un dispositif selon l'une quelconque des revendications précédentes, comprenant les étapes suivantes :

   • mesure du flux instantané et de la masse molaire instantanée d'un gaz (7) inspiré et expiré par un patient dans un courant principal (1),
   • mesure d'une concentration en $CO_2$ dans le gaz (7) inspiré et expiré par le patient dans un courant secondaire (10),
   • mesure de la masse molaire du gaz (7) inspiré et expiré par le patient dans le courant secondaire (10),
   • calcul de la concentration en $N_2$ dans le gaz (7) inspiré et expiré par le patient sur la base de la masse molaire du gaz (7) inspiré et expiré par le patient, de la concentration en $CO_2$ dans le gaz (7) inspiré et expiré par le patient et d'une valeur prédéterminée pour la concentration en vapeur d'eau en résolvant les trois équations suivantes :

$$f_{N2} + f_{O2} + f_{CO2} + f_{H2O} + f_{Ar} = 100\ \% \qquad (1)$$

$$f_{N2}M_{N2} + f_{O2}M_{O2} + f_{CO2}M_{CO2} + f_{H2O}M_{H2O} + f_{Ar}M_{Ar} = M \qquad (2)$$

$$f_{Ar} = f_{N2} \cdot \frac{0,94}{78,2} \qquad (3)$$

   dans lequel $f_x$ représente la proportion de gaz x et $M_x$ représente la masse molaire du gaz x en g/mol.

10. Procédé selon la revendication 9, **caractérisé en ce que** la concentration en $N_2$ dans le gaz (7) inspiré et expiré par le patient est multipliée par le flux mesuré, et le volume de $N_2$ inspiré et le volume de $N_2$ expiré ainsi obtenus de chaque respiration sont ajoutés à un volume total de $N_2$ expiré jusqu'à ce qu'une valeur seuil pour la concentration en $N_2$ soit atteinte.

Figur 1

Figur 2

Figur 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0597060 B1
- EP 0653919 B1
- EP 1764036 B1
- EP 1764035 A

**In der Beschreibung aufgeführte Nicht-Patentliteratur**

- **A.A. HUTCHISON ; A.C. SUM ; T.A. DEMIS ; A. ERBEN ; L.I. LANDAU.** Momentanalyse eines Multiple-Breath Stickstoff Auswaschverfahrens bei Kindern. *Am Rev Respir Dis,* 1982, vol. 125, 28-32
- Übereinkommenserklärung für Inertgas-Auswaschverfahrensmessungen bei Gebrauch von Multiple- und Single-Breath-Tests. *Eur Respir J 2013,* vol. 41, 507-522
- Übereinkommenserklärung für Inertgas-Auswaschverfahrensmessungen bei Gebrauch von Multiple- und Single Breath Tests. *Eur Respir J,* 2013, vol. 41, 507-522
- **P.M. GUSTAFSSON ; P. AURORA ; A. LINDBLAD.** Auswertung von fehlverteilter Beatmung als ein frühes Anzeichen für Lungenkrankheiten bei unter cystischen Fibrosen leidenden Kindern. *Eur Respir J,* 2003, vol. 22, 972-979
- **F. SINGER ; B. HOULTZ ; P. LATZIN ; P. ROBINSON ; P. GUSTAFSSON.** Eine realistische Validierungsstudie eines neuen Multiple-Breath-Stickstoff Auswaschverfahrens. *PloS ONE,* 2012, vol. 7 (4), e36083

- **F. SINGER et al.** Tidal volume single breath washout of two tracer gases-a practical and promising lung function test. *PLoS One,* 2011, (3), e17588
- Übereinkommenserklärung für Inertgas-Auswaschverfahrensmessungen bei Gebrauch von Multiple- und Single-Breath-Tests. *Eur Respir J,* 2013, vol. 41, 507-522
- Übereinkommenserklärung für Inertgas-Auswaschverfahrenmessungen bei Gebrauch von Multiple- und Single Breath Tests. *Eur Respir J,* 2013, vol. 41, 507-522
- **R. LAUBER ; B. SEEBERGER ; A.M. ZBINDEN.** Kohlenstoffdioxid-Analsysatoren: Genauigkeit, Alarmgrenzwerte und Auswirkungen eingreifender Gase. *Can J Anaesth,* 1995, vol. 42 (7), 643-656
- Übereinkommenserklärung für Inertgas-Auswaschverfahrenmessungen bei Gebrauch von Multiple- und Single-Breath-Tests. *Eur Respir J,* 2013, vol. 41, 507-522